# EUROPEAN PATENT SPECIFICATION

(11) **EP 0 778 751 B1**
(45) Date of publication and mention of the grant of the patent: **25.05.2005**
(21) Application number: 95932369.2
(22) Date of filing: 01.09.1995
(51) Int. Cl.: A61B 17/56, A61B 17/17

(54) **TIBIAL RESECTION METHOD AND APPARATUS**
VORRICHTUNG ZUR FEMUR- UND TIBIARRESEKTION
APPAREIL DE RESECTION DU FEMUR ET DU TIBIA

(30) Priority: 02.09.1994 US 300379; 07.06.1995 US 479363
(43) Date of publication of application: 18.06.1997
(73) Proprietor: HUDSON SURGICAL DESIGN, INC., Stewartsville, NJ 08886 (US)
(72) Inventor: GOLDSTEIN, David, B., Weehawken, NJ 07087 (US); HAINES, Timothy, G., Stewartsville, NJ 08886 (US)
(74) Representative: Needle, Jacqueline
(86) International application number: PCT/US1995/011120
(87) International publication number: WO 1996/007361

(56) References cited:
- EP-A- 0 466 659
- EP-A- 0 538 153
- EP-A- 0 682 916
- WO-A-96/01588
- FR-A- 2 664 157
- US-A- 4 524 766
- US-A- 5 053 037
- US-A- 5 147 365
- US-A- 5 306 276

## Description

Apparatus has been developed to enable a surgeon to resect the distal human femur to allow attachment of a distal femoral prosthesis (knee implant) to the human femur. Keeping in mind the ultimate goal of the procedure is to restore the knee joint to normal function, it is critical that the location and orientation of the knee implant approximates to that of the natural knee.

It is common to use the central axis of the femur, the posterior and distal femoral condyles, and/or the anterior distal femoral cortex as guides to determine the location and orientation of distal femoral resections. The location and orientation of these resections are critical in that they dictate the final location and orientation of the distal femoral implant. It is commonly thought that the location and orientation of the distal femoral implant are critical factors in the success or failure of the artificial knee joint. Past efforts have not been successful in consistently properly locating and orienting distal femoral resections.

US-A-4457307 discloses a movable saw and saw carriage which may be mounted to a patient's femur and positioned to cut the femur bone. An elongated rail is secured substantially parallel to the femur. A saw carriage and a carriage housing are attached to the rail. The saw has a blade extending substantially parallel to the direction of linear movement of the saw carriage. The saw carriage is slidably guided along paths substantially parallel to the elongated rails for making cuts in the femur bone.

US-A-4722330 describes a distal femoral surface guide for mounting on an intramedullary alignment guide for use in shaping the distal femoral surface. A conventional shaping means such as an oscillating saw or hand saw is introduced into slots in the surface guide to resect the femur.

US-A-4892093 discloses a cutting guide for a saw blade for resecting a femur. The cutting guide includes a base member having a planar base surface. A pair of laterally spaced-apart locating and securing posts are integral with the base member and project in a direction normal to the base surface to interconnect with the femur. Guide members in the form of cylindrical bars are positioned within side members attached to the base. A saw blade may be inserted between the guide surfaces to properly position the blade to cut the femur.

EP-A-0538153 shows an instrument for fitting a knee prosthesis in which various guides are fitted on a slideway.

US-A-5147365 describes apparatus which could be used for resecting a femur comprising:
positioning means for contacting a femur;
support means interconnected with the positioning means;
cutting means for cutting a distal femur;
pattern means interconnected with the support means, the pattern means comprising at least one pattern plate positionable alongside a femur; and
a cutting path described in the or each pattern plate,
wherein the cutting means is arranged to coact with the cutting path to follow a required path.

It is an object of the invention to provide an apparatus for resecting a femur.

According to the present invention, an apparatus for resecting a femur as defined above is characterised in that the cutting path described in the or each pattern plate includes a first vertical path, extending to a first diagonal path, extending to a second diagonal path, extending to a second vertical path, extending to a third diagonal path and then extending to a horizontal path, and in that the cutting means coacts with the cutting path whereby movement of the cutting means along the cutting path matches an interior profile of a distal femoral prosthesis.

In use, the pattern device is oriented and located by the use of the positioning apparatus which references the geometry of the distal femur with respect to the long axis of the femur. Once the positioning apparatus has been properly located, aligned, and initially fixed in place, the pattern device may be rigidly fixed to the distal femur. This ensures the pattern device is properly located and oriented prior to the use of the cutting device to remove material from the distal femur thus dictating the final location and orientation of the distal femoral prosthesis.

More specifically, the positioning apparatus is located and aligned utilizing the intramedullary canal of the femur, (thereby approximating the long axis of the femur). The distal surfaces of the femur, and the posterior surfaces will indicate the appropriate locations and orientations of the positioning apparatus. Fixation screws may be used to fix the guide device to the distal femur. The pattern device may then be attached to the positioning apparatus so that the location and orientation of the pattern device matches that of the positioning apparatus. Means may be present in the positioning apparatus and/or pattern device for allowing the following additional adjustments in the location and orientation of the pattern device: 1. Internal and external rotational adjustment; 2. varus and valgus angular adjustment; 3. anterior and posterior location adjustments; and 4. proximal and distal location adjustment.

Cannulated screws and fixation nails may then be used to firmly fix the pattern device to the distal femur. Thus, the location and orientation of the pattern device is established.

The pattern device possesses slot like features, or a cutting path, having geometry that closely matches the interior profile of the distal femoral prosthesis. The cutting path guides the cutting device through the aforementioned slot-like features to precisely and accurately remove material from the distal femur. Thus the distal femur is thereby properly prepared to accept a properly aligned and located distal prosthesis.

Embodiments of the present invention will hereinafter be described, by way of example, with reference to the accompanying drawings, in which:
Fig. 1 is an exploded view of a resection apparatus of the present invention showing the positioning apparatus body, the angular adjustment component and the rotational alignment component.
Fig. 2 is a side plan view of the guide device of the resection apparatus of Fig. 1 attached to a distal human femur.
Fig. 3 is an exploded view of the pattern device of the resection apparatus of the present invention.
Fig. 4 is a side plan view of the resection apparatus shown in Fig. 2 with the pattern device fixed to the distal human femur.
Fig. 5 is an exploded front view of the cutting device of the resection apparatus of the present invention.
Fig. 6 is a top plan view of the pattern device and the cutting device of the resection apparatus of the present invention affixed to the distal human femur.
Fig. 7 is a side plan view of an intramedullary rod having a helical groove for use with the resection apparatus shown in Fig. 1.

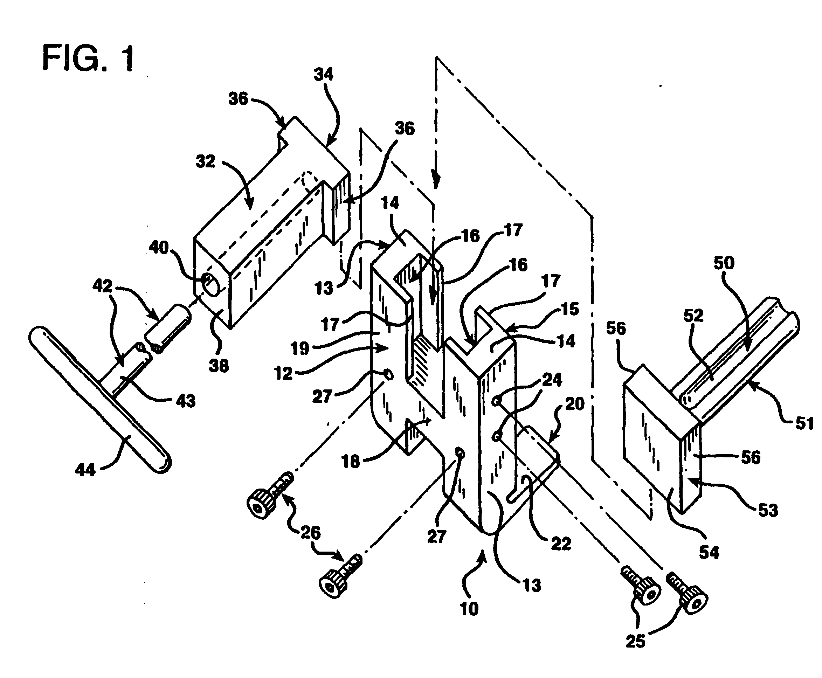

As shown generally in Fig. 1 - 6, the resecting apparatus of the present invention comprises a number of components, namely positioning apparatus generally indicated at 10 comprising a positioning body generally indicated at 12, angular adjustment block generally indicated at 32, rotational alignment device generally indicated at 50, pattern device generally indicated at 59 and cutting means generally indicated at 90.

As shown in detail in Fig. 1, the positioning apparatus, generally indicated at 10, includes a positioning body generally indicated at 12 having sides 13, top surface 14, front surface 15, back surface 19 and cross member 18. Extending from a lower end of the positioning body 12 is positioning tongue 20 having an upper surface 22. Extending into the positioning body 12 from the top surface 14 to the cross member 18 and through the front and back surfaces 15 and 19, is a gap generally defined by slots 16 and partial slot walls 17.
Sides 13 include apertures 24 for receiving locking screws 25. Also extending through the body 12 from the back surface 19 to the front surface 15 are apertures 27 for receiving fixation screws 26.

The positioning apparatus 10 receives and holds angular adjustment block generally indicated at 32. Angular adjustment block 32 includes a front surface 34 having wings 36 sized to be received by the slots 16 in the positioning body 12 to hold the angular adjustment block 32. The angular adjustment block 32 is locked into place in the positioning body 12 by means of locking screws 25 which extend through apertures 24 in the positioning body 12 and contact the wings 36 of the angular adjustment block 32 to secure the angular adjustment 32 to the positioning body 12. The angular adjustment block 32 establishes the angular alignment and anterior/posterior location of the positioning apparatus 10.

The angular adjustment block 32 also includes back surface 38 and an aperture 40 extending from the back surface 38 through the angular adjustment block 32 to the front surface 34. The aperture 40 receives an intramedullary rod 42 therethrough. The intramedullary rod 42 comprises a shaft 43 and a handle 44. The shaft 43 extends through the angular adjustment block 32 and into the intramedullary canal which extends along the axis of the femur to aid in establishing the orientation of the resection apparatus of the present invention as hereinafter described.

The rotational alignment device, generally indicated at 50, includes a shaft 51 having a groove 52 therealong and a block 53 having a back surface 54 and wings 56. The rotational alignment device 50 is interconnected with the positioning body 12 by means of the wings 56 received in slots 16 of the positioning body 12. The rotational alignment device 50 may be secured to the positioning body 12 by means of locking screws 25 which extend through apertures 24 in the positioning body 12 to contact the wings 56. The locking screws 25 may be made of various configurations depending upon their specific function. Importantly, the locking screws 25 are used to rigidly affix one component or device to another to ensure that the relative locations and orientations are maintained despite the rigors of surgery.

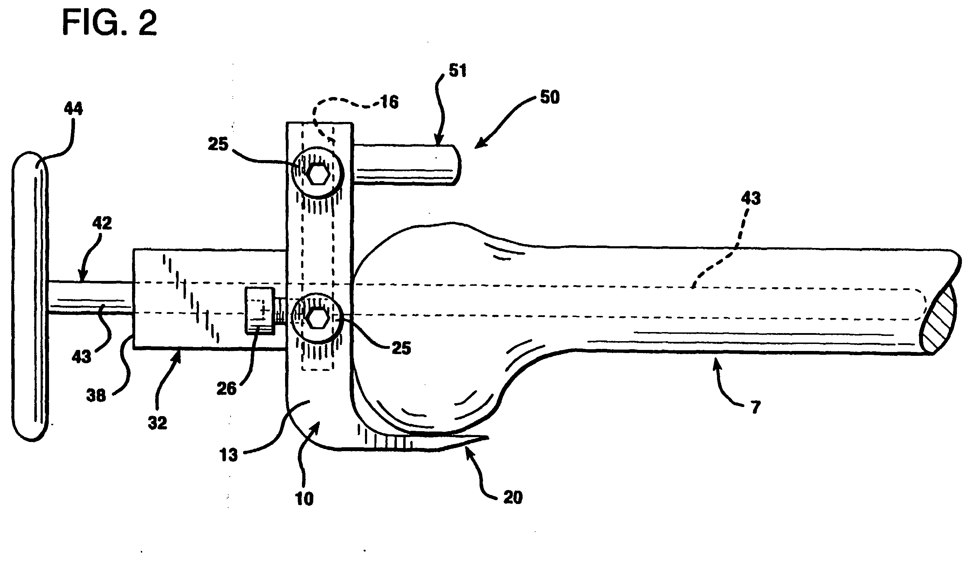

As shown in Fig. 2, wherein the positioning body 12 is fitted with the angular adjustment block 32 and the rotational alignment device 50, the entire positioning apparatus 10 is connected to a human femur 7 by means of the shaft 43 of the intramedullary rod 42. The shaft 43 extends through the angular adjustment block 32, and thereby through the positioning body 12 into the intramedullary canal which extends along the axis of the femur 7. The intramedullary rod 42, shown in Fig. 7, has a groove 41 transversing a helical path 45 along the axis of the shaft 43. The groove 41 relieves intramedullary pressure that leads to fatty embolisms. The basic concept of the intramedullary rod 42 with the groove 41, is that as it is inserted into the femur, which contains liquid fatty tissue, the liquid fatty tissue is drawn up the groove 41 of the intramedullary rod 42 to draw the fatty liquid tissue out of the femur.
Preferably, the intramedullary rod would have a hexagonal head (not shown), to permit it to be driven by a powered device such as an electrical hand held tool. Importantly, the groove 41 does not have a cutting edge, which would risk perforation of the femoral cortex. Accordingly, the device does not cut solid material, but does remove liquid material from the intramedullary canal. Therefore, the risk of fatty embolism is reduced.

After positioning body 12 is properly located against the femur 7 by means of the intramedullary rod 42 and the angular adjustment block 32, fixation screws 26 may be advanced through the apertures 27 in the positioning body 12 until they make contact with the distal femoral condyles of the femur 7, and are then driven into the distal femoral condyles of the femur 7 to initially affix the positioning apparatus to the distal femur 7. It should be noted that the fixation screws 26 may also be advanced and adjusted to make up for deficiencies in the distal femoral condyles. Accordingly, the positioning body 12 is positioned such that the front surface 15 is put into contact with the distal femoral condyles by direct contact and the tongue 20 is positioned under the femur 7 and in contact therewithin.

As can be seen in Fig. 2, the shaft 51 of the rotational alignment device 50 extends above the femur 7 and allows for rotation of the pattern device 59, hereinafter described, about the distal femur 7. Additionally, the rotational alignment device 50 allows for the anterior/posterior positioning of the pattern device 59 with respect to the femur 7. Importantly, the configurations of the positioning body 12, the angular adjustment block 32 and the rotational alignment device 50 are not limited to the structure set forth herein, but may be of different shapes and may interconnect in different ways. These components may seen be formed as a unitary or partially unitary device.

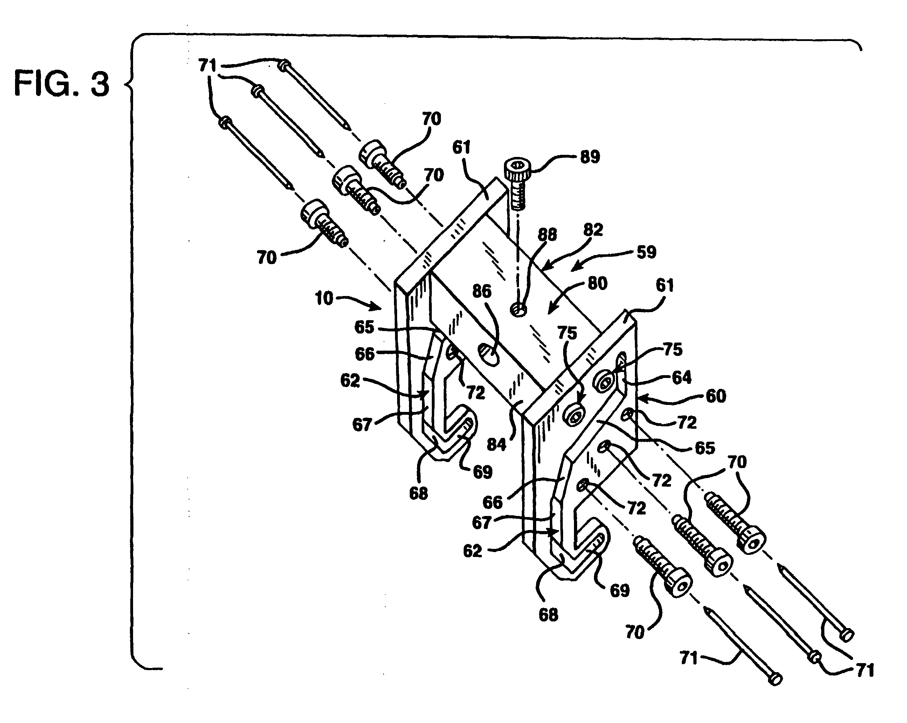

As shown in Fig. 3, the pattern device 59 includes pattern plates 60 having tops 61, and cutting paths, generally indicated at 62, extending therethrough. The cutting paths 62 outline the desired resection shape of the distal femur 7. Generally, the cutting paths 62 could include a first vertical path 64, extending to a first diagonal path 65, extending to a second diagonal path 66, extending to a second vertical path 67, extending to a third diagonal path 68 and then extending to a horizontal path 69. The pattern plates 60 also include locking screws 75 for interconnecting the pattern plates 60 with crossbar 80.

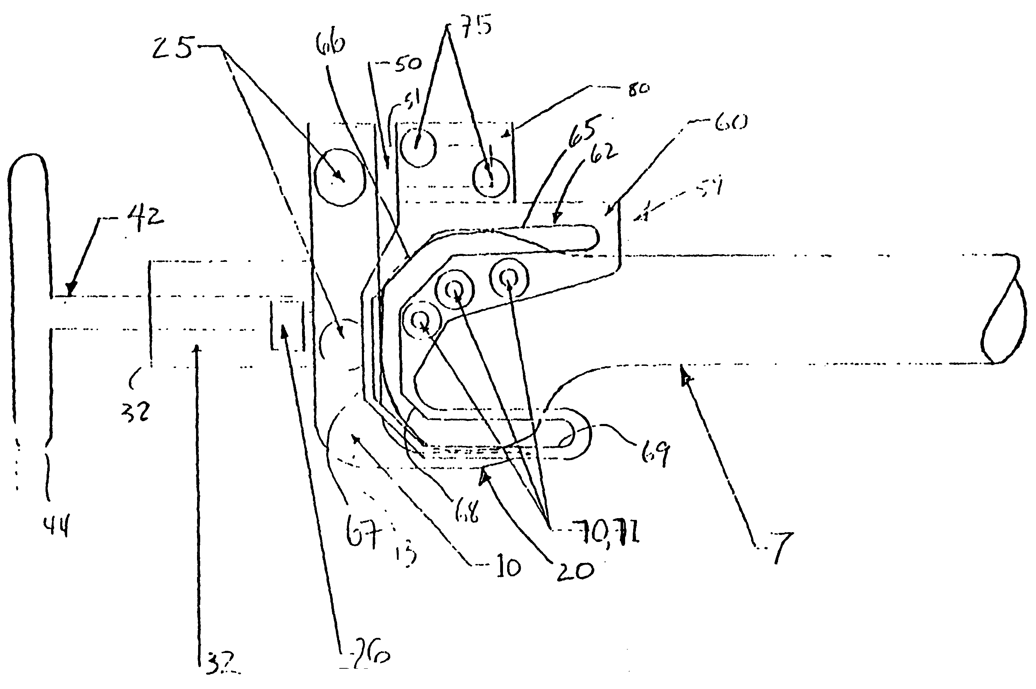

The pattern device 59 of the present invention preferably includes two pattern plates 60 held in a spaced apart relationship by crossbar 80. The crossbar 80 separates the pattern plates 60 sufficiently to permit the pattern plates 60 to extend along the sides of the distal femur 7. The crossbar 80 includes a front surface 82, back surface 84, a top surface 83, a central aperture 86 extending from the front surface 82 to the back surface 84, a lock aperture 88 extending through the top surface 83, and a lock screw 89. The central aperture 86 of the crossbar 80 receives the shaft 51 of the rotational alignment device 50. Accordingly, the pattern device 59 is interconnected with the positioning apparatus 10 so that the pattern device 59 is properly oriented with respect to the femur 7. Upon proper positioning of the crossbar 80 with respect to the shaft 51 of the rotational alignment device 50, lock screw 89 is extended through lock aperture 88 to contact the shaft 51 to lock the crossbar 80 and, accordingly, the pattern device 59, onto the shaft 51 of the rotational alignment device 50, and accordingly, to positioning apparatus 10. This completed assembly, attached to the femur 7, is shown in Fig. 4.

As additionally shown in Figs. 3 and 4, the pattern plates 60 include plate apertures 72 for receiving cannulated screws 70 which have apertures extending therethrough for receiving fixation nails 71 therethrough. Accordingly, after the pattern device 59 is interconnected with the positioning apparatus 10, and properly located and oriented with respect to femur 7, the cannulated screws 70 are extended through the plate aperture 72 to contact the sides of the distal femur 7. Then, in order to fix the pattern plates 60 with respect to the femur 7, the fixation nails 71 are driven into the distal femur 7 to lock the pattern plate 60 into position on the distal femur 7. The cannulated screws 70 have sharp leading edges for allowing decisive purchase in the distal femur 7 before the introduction of the fixation nails 71 to complete fixation of the pattern device 59 to the distal femur 7.

The pattern plates 60 by virtue of the cutting paths 62, dictate the shape of the resection of the femur 7. The cutting paths 62 are essentially channels through the pattern plates 60. The cutting paths 62 receive the cutting device and guide and guide it as it resects the surface of the distal femur 7. The pattern plates 60 straddle the distal femur 7 mediolaterally and are suspended by the crossbar 80. Likewise, the crossbar 80 maintains the proper relationship between the pattern plates 60 before and during the resection of the distal femur 7. The location of the crossbar 80 and accordingly, the pattern plates 60, may be adjusted with respect to the positioning apparatus 10 by adjusting the position of the block 53 of the rotational alignment device 50 within the slots 16 of the positioning body 12, and locking the same with locking screws 25.

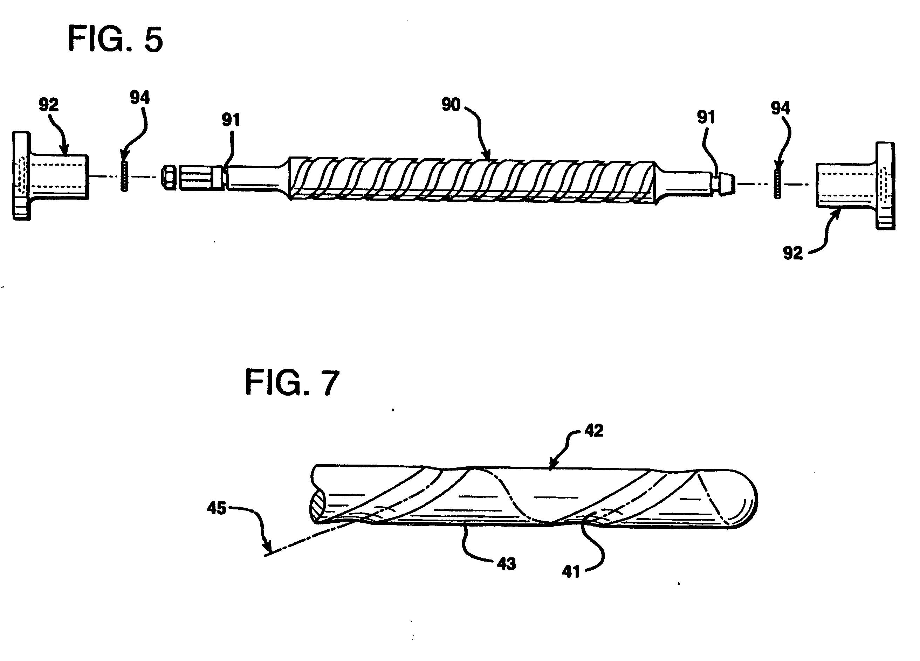

The cutting paths 62 in the pattern plates 60 receive and guide the cutting device shown in Fig. 5 and generally indicated at 90. The cutting device 90 performs the actual cutting of the femur 7 to resect the femur 7. The cutting device may be of any known configuration. In a preferred embodiment the cutting device is a drill. The drill 90 is generally cylindrical in shape and may possess helical cutting teeth along its length to cut the femur 7. The drill 90 includes a hexagonal end 95 to permit the use of an electric powered drive, typically an electric drill. Further, the drill 90 includes drill bushings 92 at the ends of the drill 90 to provide a non-metallic bearing between the cutting paths 62 in the pattern plates 60 to avoid galling and to ensure smooth articulation of the drill 90 along the cutting path 62. Positioned between the drill bushings 92 and the drill 90 are retention springs 94 which are essentially coil springs retained within the drill bushings 92 to allow the drill bushings 92 to be easily attached and removed from the drill 90. These retention springs 94 are commercially available in medical grade stainless steels. The drill bushings 92 retain the retention springs 94 which hold the drill bushings 92 in position 92 on the drill 90 while allowing the drill bushings 92 to rotate freely. The drill 90 may also include circumferential grooves 91 for allowing attachment and retention of the drill bushings 92 by means of the retention springs 94. Importantly, the configuration of the drill 90 can vary in accordance with what is known in the art as long as the cutting device can follow the cutting paths 62 in the pattern plates 60 to resect the femur 7.

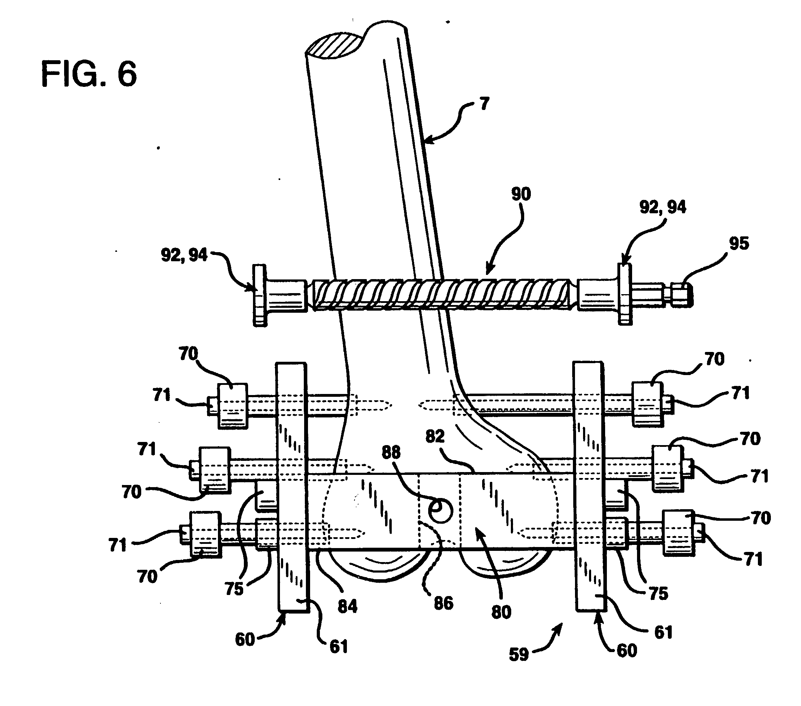

As shown in Fig. 6, after the pattern device 59 is attached to the distal femur 7, and positioned accordingly by means of the positioning apparatus 10, and secured to the distal femur 7 by means of cannulated screws 70 and fixation nails 71, positioning apparatus 10 may be removed from connection to the distal femur 7 leaving the pattern device 59 attached to the distal femur 7 to permit resecting of the distal femur. The drill 90 is then positioned within the cutting paths 62 between the pattern plates 60. Next the drill 90 is rotated by power means in connection with the hexagonal end 95, and is then moved along the cutting path 62 to resect the distal femur 7. It should also be noted that the cutting means could be operated by hand.

Instead of two pattern plates 60, a single pattern plate could be employed if it is sufficiently sturdy to support and guide the drill. The pattern plates 60 may also comprise plates having edges in the shape of the desired distal femoral resection pattern. Thus, the cutting device may be drawn along the edges of the pattern plates to resect the distal femur. Further, any cutting device that can be employed to follow the cutting paths in the pattern plates is considered to be within the scope of this invention.

The resection apparatus of the present invention, through proper use as previously described, provides extremely accurate and reproducible bone cuts. While the anterior and distal areas of the femur will almost always be able to be prepared in this manner, interference from soft tissue such as fat and ligaments may prohibit satisfactory preparation of the posterior femur. The preparation of any remaining femoral surfaces may be completed in any manner known in the art after using the instrumentation of the present invention.

It will be appreciated that modifications to, and variations in, the apparatus as described and illustrated may be made within the scope of the appended claims.

## Claims

1. An apparatus for resecting a femur comprising:
positioning means (10) for contacting a femur;
support means (50) interconnected with positioning means (10);
cutting means (90) for cutting a distal femur;
pattern means (59) interconnected with the support means (50), the pattern means comprising at least one pattern plate (60) positionable alongside a femur; and
a cutting path (62) described in the or each pattern plate,
wherein the cutting means (90) is arranged to coact with the cutting path (62) to follow a required path;
the apparatus being **characterised in that** the cutting path (62) described in the or each pattern plate (60) includes a first vertical path (64), extending to a first diagonal path (65), extending to a second diagonal path (66), extending to a second vertical path (67), extending to a third diagonal path (68) and then extending to a horizontal path (69), and
**in that** the cutting means (90) coacts with the cutting path (62) whereby movement of the cutting means (90) along the cutting path (62) matches an interior profile of a distal femoral prosthesis.

2. An apparatus as claimed in Claim 1, further comprising an intramedullary rod (42) insertable into a femur, the intramedullary rod interconnected with the positioning means (10) to align the positioning means with respect to a femur.

3. An apparatus as claimed in Claim 2, wherein the intramedullary rod (42) includes at least one groove (41) extending helically along the length of the intramedullary rod.

4. An apparatus as claimed in Claim 2 or Claim 3, further comprising adjustment means (32) for receiving the intramedullary rod, the adjustment means being interconnected with the positioning means (10).

5. An apparatus as claimed in Claim 4, wherein the positioning means (10) further comprises a channel (16) extending into the positioning means (10) from an upper surface thereof.

6. An apparatus as claimed in Claim 5, wherein the adjustment means further comprises wings (36) sized to be received by the channel (16) in the positioning means (10).

7. An apparatus as claimed in any preceding claim, wherein the cutting means (90) comprises a cylindrical drill extending through the cutting path (62) and movable along the cutting path (62) after the positioning means (10) and the adjustment means (32) are removed from a femur.

8. An apparatus as claimed in any preceding claim, wherein the pattern means (59) comprises two opposing pattern plates (60) which are spaced apart and extend substantially parallel to one another and are positionable on either side of a femur to straddle the femur, wherein the respective cutting paths (62) described in each of the two opposed pattern plates (60) are substantially identical, and wherein the cutting means (90) spans the two pattern plates (60) and coacts with both cutting paths (62).

9. An apparatus as claimed in Claim 8, wherein the two pattern plates (60) are held in the spaced apart relationship by a crossbar (80), and wherein a central aperture (86) of the crossbar receives a shaft (51) of a rotational alignment device (50) supported by said positioning means (10).

10. An apparatus as claimed in any preceding claim, further comprising fixing means (70,71) for fixing the pattern means (59) to a distal femur.

11. An apparatus as claimed in Claim 10, wherein the fixing means comprises cannulated screws (70) extended through apertures in the or each pattern plate (60) and fixation nails (71) extendable through the cannulated screws into a distal femur.

12. An apparatus as claimed in any preceding claim, wherein the positioning means (10) is arranged to position the resecting apparatus on a distal human femur, the positioning means having a positioning body (12) comprising:
a front surface (15) for contacting a human femur;
a tongue (20) extending from a lower end of the positioning body for extending under a human femur;
attachment means (26) for attaching the positioning body to a distal human femur,
the apparatus further comprising:
angular adjustment means (32) for adjusting the angle of the positioning means (10), the angular adjustment means comprising:
an adjustment body (32);
a rod (42) extending through the adjustment body and into the distal human femur; and
attachment means (16,36) for attaching the angular adjustment means (32) to the positioning means (10); and
the apparatus further comprising:
rotational alignment means (50) comprising:
an alignment body (50);
a shaft (51) extending from the alignment body; and
attachment means (53,16) for attaching the rotational alignment means to the positioning means (10).

13. An apparatus as claimed in Claim 12, wherein the body (12) of the positioning means (10) further comprises a channel (16) extending into the positioning body from a top surface of the positioning body.

14. An apparatus as claimed in Claim 13, wherein the body of the angular adjustment means (32) further includes wings (36) sized to be received by the channel (16) in the body (12) of the positioning means.

15. An apparatus as claimed in Claim 13 or Claim 14, wherein the body of the rotational alignment means (50) further includes wings (53) sized to be received by the channel (16) in the body of the positioning means (10).

## Patentansprüche

1. Vorrichtung zur Resektion eines Femurs, die aufweist:
eine Positioniereinrichtung (10) zum Kontaktieren eines Femurs;
eine Stützeinrichtung (50), die mit der Positioniereinrichtung (10) verbunden ist;
eine Schneideinrichtung (90) zum Schneiden eines distalen Femurs;
eine Lehre (59), die mit der Stützeinrichtung (50) verbunden ist, wobei die Lehre mindestens eine Schablone (60) aufweist, die entlang eines Femurs angeordnet werden kann; und
einen Schneidweg (62), der in der oder jeder Schablone beschrieben wird; wobei die Schneideinrichtung (90) so ausgebildet ist, dass sie mit dem Schneidweg (62) zusammenwirkt, um einem vorgeschriebenen Weg zu folgen;
wobei die Vorrichtung **dadurch gekennzeichnet ist, dass** der in der oder jeder Schablone (60) beschriebene Schneidweg (62) einen ersten vertikalen Weg (64) beinhaltet, der sich zu einem ersten diagonalen Weg (65) erstreckt, der sich zu einem zweiten diagonalen Weg (66) erstreckt, der sich zu einem zweiten vertikalen Weg (67) erstreckt, der sich zu einem dritten diagonalen Weg (68) und dann zu einem horizontalen Weg (69) erstreckt; und
dass die Schneideinrichtung (90) mit dem Schneidweg (62) zusammenwirkt, wodurch eine Bewegung der Schneideinrichtung (90) entlang des Schneidweges (62) einem Innenprofil einer distalen Femurprothese entspricht.

2. Vorrichtung nach Anspruch 1, die weiterhin einen intramedullären Stab (42) aufweist, der in ein Femur eingeführt werden kann, wobei der intramedulläre Stab mit der Positioniereinrichtung (10) verbunden ist, um die Positioniereinrichtung in Bezug auf ein Femur auszurichten.

3. Vorrichtung nach Anspruch 2, wobei der intramedulläre Stab (42) mindestens eine Nut (41) beinhaltet, die sich schraubenförmig entlang der Länge des intramedullären Stabes erstreckt.

4. Vorrichtung nach Anspruch 2 oder 3, die weiterhin eine Verstelleinrichtung (32) zur Aufnahme der intramedullären Stabes aufweist, wobei die Verstelleinrichtung mit der Positioniereinrichtung (10) verbunden ist.

5. Vorrichtung nach Anspruch 4, wobei die Positioniereinrichtung (10) weiterhin einen Kanal (16) aufweist, der sich von einer oberen Fläche der Positioniereinrichtung (10) in diese erstreckt.

6. Vorrichtung nach Anspruch 5, wobei die Verstelleinrichtung weiterhin Flügel (36) aufweist, die so bemessen sind, dass sie von dem Kanal (16) in der Positioniereinrichtung (10) aufgenommen werden können.

7. Vorrichtung nach einem der vorhergehenden Ansprüche, wobei die Schneideinrichtung (90) einen zylindrischen Bohrer aufweist, der sich durch den Schneidweg (62) erstreckt und entlang des Schneidweges (62) beweglich ist, nachdem die Positioniereinrichtung (10) und die Verstelleinrichtung (32) von einem Femur entfernt wurden.

8. Vorrichtung nach einem der vorhergehenden Ansprüche, wobei die Lehre (59) zwei gegenüberliegende Schablonen (60) aufweist, die voneinander beabstandet sind und sich im Wesentlichen parallel zueinander erstrecken und auf jeder Seite eines Femurs angeordnet werden können, so dass sie beiderseits des Femurs liegen, wobei die jeweiligen Schneidwege (62), die in jeder der zwei gegenüberliegenden Schablonen (60) beschrieben sind, im Wesentlichen identisch sind, und wobei die Schneideinrichtung (90) sich über die zwei Schablonen (60) erstreckt und mit beiden Schneidwegen (62) zusammenwirkt.

9. Vorrichtung nach Anspruch 8, wobei die zwei Schablonen (60) durch einen Querbalken (80) in einem voneinander beabstandeten Verhältnis gehalten sind, und wobei eine zentrale Apertur (86) des Querbalkens einen Schaft (51) einer Dreh-Ausrichtevorrichtung (50) aufnimmt, die von der Positioniereinrichtung (10) getragen wird.

10. Vorrichtung nach einem der vorhergehenden Ansprüche, die weiterhin eine Fixationseinrichtung (70, 71) zum Fixieren der Lehre (59) an einem distalen Femur aufweist.

11. Vorrichtung nach Anspruch 10, wobei die Fixationseinrichtung kannulierte Schrauben (70) aufweist, die sich durch Aperturen in der oder jeder Schablone (60) erstrecken, und Fixationsnägel (71), die sich durch die kannulierten Schrauben in ein distales Femur erstrecken können.

12. Vorrichtung nach einem der vorhergehenden Ansprüche, wobei die Positioniereinrichtung (10) so angeordnet ist, dass sie die Resektionsvorrichtung an einem distalen menschlichen Femur positioniert, wobei die Positioniereinrichtung einen Positionierkörper (12) hat, welcher aufweist:
eine Vorderfläche (15) zum Kontaktieren eines menschlichen Femurs;
eine Zunge (20), die sich von einem unteren Ende des Positionierkörpers zur Erstreckung unter einem menschlichen Femur erstreckt;
eine Befestigungseinrichtung (26) zum Befestigen des Positionierkörpers an einem distalen menschlichen Femur;
wobei die Vorrichtung weiter aufweist:
eine Winkel-Verstelleinrichtung (32) zum Verstellen des Winkels der Positioniereinrichtung (10), wobei die Winkel-Verstelleinrichtung aufweist:
einen Verstellkörper (32);
einen Stab (42), der sich durch den Verstellkörper und in das distale menschliche Femur erstreckt; und
eine Befestigungseinrichtung (16, 36) zum Befestigen der Winkel-Verstelleinrichtung (32) an der Positioniereinrichtung (10); und
die Vorrichtung weiterhin aufweist:
eine Dreh-Ausrichteeinrichtung (50), die aufweist:
einen Ausrichtekörper (50);
einen Schaft (51), der sich von dem Ausrichtekörper erstreckt; und eine Befestigungseinrichtung (53, 16) zum Befestigen der Dreh-Ausrichteeinrichtung an der Positioniereinrichtung (10).

13. Vorrichtung nach Anspruch 12, wobei der Körper (12) der Positioniereinrichtung (10) weiterhin einen Kanal (16) aufweist, der sich in den Positionierkörper von einer oberen Fläche des Positionierkörpers erstreckt.

14. Vorrichtung nach Anspruch 13, wobei der Körper der Winkel-Verstelleinrichtung (32) weiterhin Flügel (36) aufweist, die so bemessen sind, dass sie von dem Kanal (16) in dem Körper (12) der Positioniereinrichtung aufgenommen werden können.

15. Vorrichtung nach Anspruch 13 oder 14, wobei der Körper der Dreh-Ausrichteeinrichtung (50) weiterhin Flügel (53) beinhaltet, die so bemessen sind, dass sie von dem Kanal (16) in dem Körper der Positioniereinrichtung (10) aufgenommen werden können.

## Revendications

1. Appareil de résection d'un fémur comprenant :
des moyens de positionnement (10) destinés à être mis en contact avec un fémur ;
des moyens de support (50) jumelés avec les moyens de positionnement (10) ;
des moyens de coupe (90) destinés à couper un fémur distal ;
des moyens de guidage (59) jumelés avec les moyens de support (50), les moyens de guidage comprenant au moins une plaque de guidage (60) positionnable le long d'un fémur, et
une trajectoire de coupe (62) décrite dans la ou dans chaque plaque de guidage,
dans lequel les moyens de coupe (90) sont agencés de façon à coagir avec la trajectoire de coupe (62) pour suivre une trajectoire requise ;
l'appareil étant **caractérisé en ce que** la trajectoire de coupe (62) définie dans la ou dans chaque plaque de guidage (60) comprend une première trajectoire verticale (64), s'étendant vers une première trajectoire diagonale (65), s'étendant vers une deuxième trajectoire diagonale (66), s'étendant vers une seconde trajectoire verticale (67), s'étendant vers une troisième trajectoire diagonale (68) puis s'étendant vers une trajectoire horizontale (69), et
**en ce que** les moyens de coupe (90) coagissent avec la trajectoire de coupe (62) moyennant quoi le mouvement des moyens de coupe (90) le long de la trajectoire de coupe (62) correspond à un profil interne d'une prothèse de fémur distal.

2. Appareil selon la revendication 1, comprenant en outre une tige centromédullaire (42) pouvant être insérée dans un fémur, la tige centromédullaire étant jumelée avec les moyens de positionnement (10) pour aligner les moyens de positionnement (10) avec un fémur.

3. Appareil selon la revendication 2, dans lequel la tige centromédullaire (42) comprend au moins une rainure (41) s'étendant de façon hélicoïdale sur toute la longueur de la tige centromédullaire.

4. Appareil selon la revendication 2 ou 3, comprenant en outre des moyens d'ajustement (32) destinés à recevoir la tige centromédullaire, les moyens d'ajustement étant jumelés avec les moyens de positionnement (10).

5. Appareil selon la revendication 4, dans lequel les moyens de positionnement (10) comprennent en outre une gouttière (16) s'étendant dans les moyens de positionnement (10) à partir d'une surface supérieure de ceux-ci.

6. Appareil selon la revendication 5, dans lequel les moyens d'ajustement comprennent en outre des ailettes (36) dimensionnées pour être reçues par la gouttière (16) présente dans les moyens de positionnement (10).

7. Appareil selon l'une quelconque des revendications précédentes, dans lequel les moyens de coupe (90) comprennent une fraise cylindrique s'étendant à travers la trajectoire de coupe (62) et déplaçable le long de la trajectoire de coupe (62) après que les moyens de positionnement (10) et les moyens d'ajustement (32) aient été enlevés d'un fémur.

8. Appareil selon l'une quelconque des revendications précédentes, dans lequel les moyens de guidage (59) comprennent deux plaques de guidage opposées (60) qui sont séparées et s'étendent sensiblement parallèlement l'une à l'autre et sont positionnables sur l'un ou l'autre côté d'un fémur pour passer de part et d'autre du fémur, dans lequel les trajectoires de coupe (62) respectives définies dans chacune des deux plaques de guidage opposées (60) sont sensiblement identiques, et dans lequel les moyens de coupe (90) couvrent les deux plaques de guidage (60) et coagissent avec les deux trajectoires de coupe (62).

9. Appareil selon la revendication 8, dans lequel les deux plaques de guidage (60) sont maintenues séparées par une barre transversale (80) et dans lequel une ouverture centrale (86) de la barre transversale reçoit un arbre (51) d'un dispositif d'alignement rotatif (50) supporté par lesdits moyens de positionnement (10).

10. Appareil selon l'une quelconque des revendications précédentes, comprenant en outre des moyens de fixation (70, 71) destinés à fixer les moyens de guidage (59) à un fémur distal.

11. Appareil selon la revendication 10, dans lequel les moyens de fixation comprennent des vis tubulaires (70) qui s'étendent à travers des ouvertures de la ou de chaque plaque de guidage (60) et des clous de fixation (71) pouvant s'étendre à travers les vis tubulaires dans un fémur distal.

12. Appareil selon l'une quelconque des revendications précédentes, dans lequel les moyens de positionnement (10) sont agencés de façon à positionner l'appareil de résection sur un fémur distal humain, les moyens de positionnement présentant un corps de positionnement (12) comprenant :
une surface avant (15) destinée à être mise en contact avec un fémur humain ;
une languette (20) s'étendant à partir d'une extrémité inférieure du corps de positionnement destinée à passer sous un fémur humain ;
des moyens d'attache (26) destinés à attacher le corps de positionnement à un fémur distal humain ;
l'appareil comprenant en outre :
des moyens d'ajustement angulaire (32) destinés à ajuster l'angle des moyens de positionnement (10), les moyens d'ajustement angulaire comprenant :
un corps d'ajustement (32) ;
une tige (42) s'étendant à travers le corps d'ajustement et dans le fémur distal humain ; et
des moyens d'attache (16, 36) destinés à attacher les moyens d'ajustement angulaire (32) aux moyens de positionnement (10) ; et
l'appareil comprenant en outre :
des moyens d'alignement rotatif (50) comprenant :
un corps d'alignement (50) ;
un arbre (51) s'étendant depuis le corps d'alignement ; et
des moyens d'attache (53, 16) destinés à attacher les moyens d'alignement rotatif aux moyens de positionnement (10).

13. Appareil selon la revendication 12, dans lequel le corps (12) des moyens de positionnement (10) comprend en outre une gouttière (16) s'étendant dans le corps de positionnement à partir d'une surface supérieure du corps de positionnement.

14. Appareil selon la revendication 13, dans lequel le corps des moyens d'ajustement angulaire (32) comprend en outre des ailettes (36) dimensionnées pour être reçues par la gouttière (16) dans le corps (12) des moyens de positionnement.

15. Appareil selon la revendication 13 ou 14, dans lequel le corps des moyens d'alignement rotatif (50) comprend en outre des ailettes (53) dimensionnées pour être reçues par la gouttière (16) dans le corps des moyens de positionnement (10).
